# EUROPÄISCHE PATENTSCHRIFT

(11) **EP 1 776 582 B1**
(45) Veröffentlichungstag und Bekanntmachung des Hinweises auf die Patenterteilung: **24.06.2020**
(21) Anmeldenummer: 05777490.3
(22) Anmeldetag: 29.07.2005
(51) Int. Cl.: G01N 33/48, G01N 37/00, A61M 1/34, A61M 1/36, A61M 1/38

(54) **VORRICHTUNG UND VERFAHREN ZUM ISOLIEREN VON ZELLEN, BIOPARTIKELN UND/ODER MOLEKÜLEN AUS FLÜSSIGKEITEN MIT DEM ZIEL EINER ANWENDUNG FÜR TIERE, IN DER BIOTECHNOLOGIE (EINSCHLIESSLICH DER BIOLOGISCHEN FORSCHUNG) UND DER MEDIZINISCHEN DIAGNOSTIK**
DEVICE AND METHOD FOR ISOLATING CELLS, BIOPARTICLES AND/OR MOLECULES FROM LIQUIDS FOR USE WITH ANIMALS, IN BIOTECHNOLOGY, (INCLUDING BIOTECHNOLOGICAL RESEARCH) AND MEDICAL DIAGNOSTICS
PROCEDE ET DISPOSITIF POUR ISOLER DES CELLULES, DES BIOPARTICULES ET/OU DES MOLECULES DE LIQUIDES POUR UNE UTILISATION POUR LES ANIMAUX, EN BIOTECHNOLOGIE (Y COMPRIS LA RECHERCHE BIOLOGIQUE) ET POUR LE DIAGNOSTIC MEDICAL

(30) Priorität: 30.07.2004 DE 102004037476
(43) Veröffentlichungstag der Anmeldung: 25.04.2007
(73) Patentinhaber: pluriSelect Life Sciences UG & Co. KG, 04103 Leipzig (DE)
(72) Erfinder: HEINRICH, Hans-Werner, 17489 Greifswald (DE)
(74) Vertreter: Baumbach, Friedrich
(86) Internationale Anmeldenummer: PCT/DE2005/001374
(87) Internationale Veröffentlichungsnummer: WO 2006/012886

(56) Entgegenhaltungen:
- US-A- 6 039 946
- AMEER GUILLERMO A ET AL: "A novel immunoadsorption device for removing beta2-microglobulin from whole blood" KIDNEY INTERNATIONAL, Bd. 59, Nr. 4, April 2001 (2001-04), Seiten 1544-1550, XP002358719 ISSN: 0085-2538
- AMEER G A ET AL: "Regional heparinization via simultaneous separation and reaction in a novel Taylor-Couette flow device" BIOTECHNOL BIOENG; BIOTECHNOLOGY AND BIOENGINEERING 1999 JOHN WILEY & SONS INC, NEW YORK, NY, USA, Bd. 63, Nr. 5, 1999, Seiten 618-624, XP002358720
- KUNKEL SIEGFRIED ET AL: "Selective removal of circulating immune complexes from patient plasma" ARTIFICIAL ORGANS, Bd. 26, Nr. 2, Februar 2002 (2002-02), Seiten 124-132, XP002358721 ISSN: 0160-564X
- RYE ET AL: "Immunobead filtration: A novel approach for the isolation and propagation of tumor cells", AMERICAN JOURNAL OF PATHOLOGY; [10640], ELSEVIER INC, US, vol. 150, no. 1, 1 January 1997 (1997-01-01), pages 99-106, XP002122421, ISSN: 0002-9440

## Beschreibung

Die Erfindung beschreibt eine Vorrichtung und ein Verfahren zum Isolieren von Zellen aus Blut. Mit ihr können unter Verwendung geeigneter Träger und bekannter Immobilisierungsverfahren spezifische Zellen erkannt und abgetrennt werden. Anwendungsgebiete der Erfindung sind Tiere, die Biotechnologie (einschließlich der biologischen Forschung) und die medizinische Diagnostik.

Die Isolierung von Zellen aus Blut ist auf vielen technischen Gebieten von Bedeutung, zahlreiche Verfahren sind dazu bekannt.

Die Zellanalyse und die Zellseparation werden seit Jahrzehnten mittels Fluorescence Activated Cell Sorting (FACS) vorgenommen. Es ist die Methode der Wahl für die Analytik spezifischer Zellpopulationen durch Oberflächenmarker. Probleme bereitet die FACS-Anwendung für die Isolierung großer Zellzahlen. Das die Zellen enthaltende Medium muss stark verdünnt werden, die Trennzeit ist relativ lange für größere Zellmengen und es bereitet Probleme, aseptische Bedingungen einzuhalten. Das Verfahren verursacht insgesamt erhebliche Kosten.

Zur Erkennung und Isolierung von Zellen werden seit mehr als 10 Jahren im wachsenden Maße magnetische Trennverfahren eingesetzt. Dazu werden entweder die Fängermoleküle mit Eisen beladen oder einen Eisenkern enthaltende Mikropartikel werden mit dem Fängermolekül beschichtet. Die Abtrennung erfolgt durch ein starkes magnetisches Feld. Die immunmagnetische Separation, erfolgreich kommerzialisiert u. a. durch Dynal und Miltenyi Biotec, hat sich als einfache, relativ kostengünstige Methode der Zelltrennung etabliert. Besonders im Vergleich zur Fow-Zytometrie hat sich die magnetische Separation für die Isolierung von relativ seltenen Zelltypen bewährt, so z. B. für die Isolierung fetaler Zellen aus dem mütterlichen Blut für die pränatale Diagnostik. Eine weitere Anwendung ist der Nachweis von Tumorzellen im Blut nach chirurgischer Entfernung des Primärtumors zur Einleitung weiterer Behandlungen.

Für therapeutische Zwecke werden heute routinemäßig syngene CD34⁺-periphere Blutstammzellen von Patienten, die an bestimmten malignen Erkrankungen des Blut bildenden Systems leiden, für eine Reimplantation gewonnen. Das geschieht vorzugsweise durch die Aufreinigung von Leukozyten mit spezifischen Antikörpern, die an Magnetic Beads gekoppelt sind. Für die Zellfraktionierung während der Blut/Zell-Spende stehen mehrere Systeme zur Verfügung, die die unterschiedliche Größe und spezifische Dichte der Blutzellen für eine Trennung im Schwerefeld (Zentrifugation) nutzen.

Ein Nachteil aller Sortierungsverfahren ist, dass sie nicht kontinuierlich durchgeführt werden können. D. h. es wird eine Blut- bzw. Lymphozyten-Probe entnommen, die Zellen werden mit den immunmagnetischen Partikeln inkubiert. Nach dem Abtrennen und Waschen werden die Zellen vom Magnetpartikel abgelöst und können dann für therapeutische Zwecke eingesetzt werden. Ein guter Überblick über FACS und MACS wird gegeben in "Fow Cytometry and Sorting" (Ed. Melamed et al., Wiley & Sons, Inc., New York, 1990).

Andere Methoden der Isolierung und/oder Entfernung von Zellen sind beschrieben in EP 12311956A2, US 6900029, US 6432630, US 20020012953A1, DE 10022635A1, US 5246829, US 5739033, US 5763203, EP 0016552A1, WO 00/38762, EP 0502213B1 und EP 0554460B1.

Der Erfindung liegt die Aufgabe zugrunde, ein einfaches System zur Abtrennung von Zellen, Biopartikeln und anderen Molekülen zu entwickeln, das für Tiere angewendet werden kann, ebenso in der Biotechnologie einschließlich der biologischen Forschung und in der medizinischen Diagnostik.

Die Erfindung wird gemäß Anspruch 1 realisiert, die Unteransprüche 2 - 12 sind Vorzugsvarianten.

Die Erfindung beschreibt ein einfaches Trennverfahren, das überall dort angewendet werden kann, wo für das zu separierende Agens eines Stoffgemisches ein spezifischer Ligand funktionsfähig an eine Oberfläche immobilisiert werden kann. Die eigentliche Separation erfolgt durch Sortierung nach Partikelgröße (Filtration, Sieben).

Für die Lösung dieser Aufgabe werden Standard-Verfahren der Kopplung spezifischer Liganden an einen festen Träger genutzt.

Als feste Träger kommen bekannte Biopolymere wie Membranen oder Partikel aus organischen oder synthetischen Polymeren zum Einsatz. Die Oberfläche kann biokompatibel sein und bestehen aus Kollagen, gereinigten Proteinen, gereinigten Peptiden, Polysacchariden wie z.B. Chitosan, Alginat, Dextran, Zellulose, Glycosaminoglycanen oder synthetische Polymere wie Polystyren, Polyester, Polyäther, Polyanhydride, Polyalkylcyanoacrylate, Polyacrylamide, Polyorthoester, Polyvinylacetate, Blockcopolymere, Polypropyle, Polytetrafluoroäthylen (PTFE) oder Polyurethan. Darüber hinaus können die Polymere Milchsäure-Polymere oder Copolymere (Milchsäure und/oder Glykol- Säure (PLGA) enthalten.

Die verwendeten Oberflächen können biodegradierbar oder nicht-biodegradierbar sein.

Die benutzten spezifischen Liganden für die Bindung der Ziel-Moleküle auf der Oberfläche von Zellen können natürlich oder synthetischer Natur sein, so z. B. Antikörper, Antikörperfragmente, Peptide, Polypeptide, Glykopeptide, lösliche Rezeptoren, Steroide, Hormone, Mitogene, Antigene, Superantigene, Wachstumsfaktoren, Cytokine, Leptine, Virusproteine, Adhäsionsmoleküle oder Chemokine.

Für die spezifische Verwendung wird mindestens ein Antikörper oder ein Antikörperfragment eingesetzt, wobei die spezifischen Liganden kovalent an diese gebunden oder über Spacer oder Linker an sie fixiert sind.

Die Trägersubstanz kann jegliche Geometrie aufweisen. Membranen als Kapillare oder Partikel bieten den Vorteil einer großen Oberfläche. Für das zu beschreibende Verfahren werden Mikropartikel mit einem Durchmesser von >10 µm < 800µm, vorzugsweise 50 - 500µm eingesetzt. Für andere Aufgabenstellungen können aber auch größere oder kleinere Partikel eingesetzt werden.

Die mit spezifischen Liganden (z.B. Antikörper gegen CD34 oder CD1d, oder gegen HIV-gp120) aktivierten Mikropartikel werden mit entnommenem Blut, das mit üblichen vorzugsweise 50 - 500µm eingesetzt. Für andere Aufgabenstellungen können aber auch größere oder kleinere Partikel eingesetzt werden.

Die mit spezifischen Liganden (z.B. Antikörper gegen CD34 oder CD1d, oder gegen HIV-gp120) aktivierten Mikropartikel werden mit entnommenem Blut, das mit üblichen Antikoagulantien behandelt wird, ex-vivo in Kontakt gebracht. Das geschieht in einem besonderen Reaktionsraum. Soll der Reaktionsraum in einen extra-korporalen Kreislauf verbracht werden, sichern Schlauchverbindungen, Ventile, Filter und Pumpen, verbunden mit der Sicherungstechnik, dass das System ohne Nebenwirkungen für den Patienten nach entsprechender Antikoagulation betrieben werden kann.

Die Zielzellen werden durch die funktionalisierten Mikropartikel gebunden. Die Abtrennung der nun beladenen Mikropartikel geschieht durch hydrostatischen Druck unter Verwendung einer Membran (Siebe), vorzugsweise in Form einer Röhre, die alle Blutbestandteile ungehindert durchtreten lässt (Porengröße >10µm <800µm) aber die Mikropartikel zurückhält. Dabei kann die Filtration sowohl durch vertikale wie auch tangentiale Druckeinwirkung erfolgen. Die im Lumen verbleibenden Partikel werden in das Reaktionsgefäß zurückgeführt oder für analytische oder präparative Zwecke abgeleitet.

Nach Unterbrechung der Blutzufuhr kann das Reaktionsgefäß für die Ablösung der Zellen von den Mikropartikeln genutzt werden. Die so vereinzelten, dispergierten Zellen können mittels gleichen Verfahrens von den Partikeln abgetrennt werden und stehen nun für diagnostische Anwendungen zur Verfügung.

Eine weitere Anwendung ist die gezielte Isolierung verschiedener Zelltypen mit dem Ziel der Co-Kultivation, immer dann, wenn die jeweiligen Zelltypen Oberflächen-Moleküle und/oder Biomoleküle wie Cytokine anderer Zelltypen benötigen, um ihre gewünschte Funktion zu erfüllen.

Um für den notwendigen Zell-Zell-Kontakt eine ausreichende Nähe der gewünschten Zielzellen zu erreichen, können mehrere spezifische Liganden auf einem Partikel gebunden sein.

Spezifische Zellen für diese Anwendung können u. a. T-Zellen, B-Zellen oder Stammzellen sein.

Das Verfahren kann sowohl diskontinuierlich wie auch kontinuierlich durchgeführt werden. Aus der hohen Variabilität in der Partikelgestaltung hinsichtlich Material, Durchmesser und Oberflächenmodifikationen ergibt sich eine riesige Vielfalt von Anwendungsmöglichkeiten bei Tieren, in der medizinischen Diagnostik, aber auch in der Biotechnologie und der biologischen Forschung

### Ausführungsbeispiel 1 :

### Isolierung von CD4-positiven Zellen aus Rattenvollblut

Ascitis Antikörper (RIB 5/2) wurden mit Hilfe vom Millipore Montage Antibody Purification Kit (LSK2 ABG 20) nach Protokoll durchgeführt.

Die Aufreinigung wurde anschließend mit einem SDS Denaturierungs-Gel (10%) überprüft.

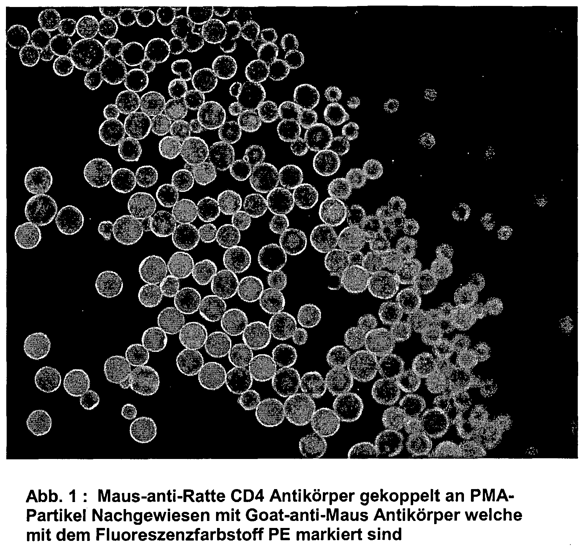

Kopplung der Anti-CD4 Antikörper an die Polymethylmethcrylat (PMA)-Partikel
1. 1 ml PMA (Partikeldurchmesser = 40 µm +/- 10 µm; 10 mg/ml; COOH/PEG-COOH modifiziert,) zentrifugiert 2 min 3.000x g, Überstand verwerfen und in 1 ml 0,1M MES Puffer pH 6.3 aufnehmen.
2. 2 mg EDC und 2,4 mg N-Hydroxysuccinimid in 0,5 ml 0,1M MES Puffer pH 6,3 lösen und zu der PMA-Partikel-Suspension geben. 1 Stunde bei Raumtemperatur unter Rühren inkubieren (Aktivierung der Partikel).
3. PMA-Partikel separieren durch Zentrifugation und 2mal waschen mit 0,1M MES Puffer pH 6,3.
4. Aktivierte PMA-Partikel in 1 ml 0,1M MES Puffer pH 6.3 aufnehmen mit 100-150 µg Antikörper; Kopplungsreaktion bei Raumtemperatur für 16 Stunden (über Nacht).
5. Neutralisation freier Bindungsstellen durch Zugabe von 100 µl 1M Ethanolamine mit anschließender Inkubation für 1 Stunde.
6. Separieren mittels Zentrifugation und waschen der funktionalisierten PMA-Partikel mit PBS 3 mal.
7. Aufnahme der anti-CD4-PMA-Partikel in 1 ml PBS pH 7,4 und Lagerung bei 4°C. Überprüfung der Antikörperkopplung mittels Goat-anti-Maus Antikörper PE markiert (Abbildung 1).

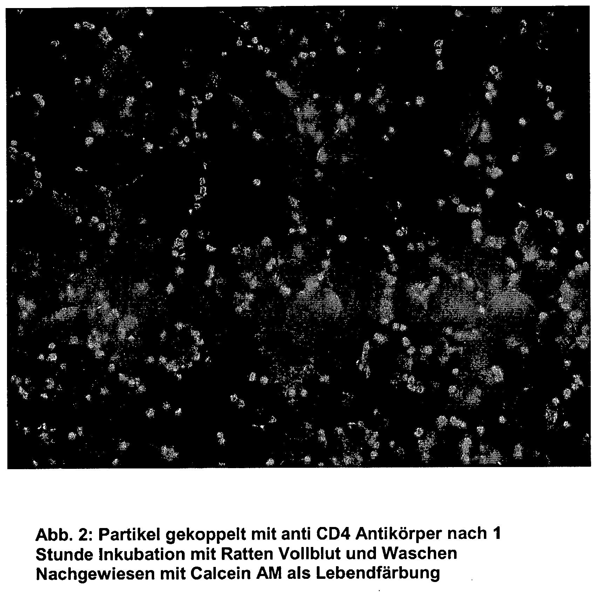

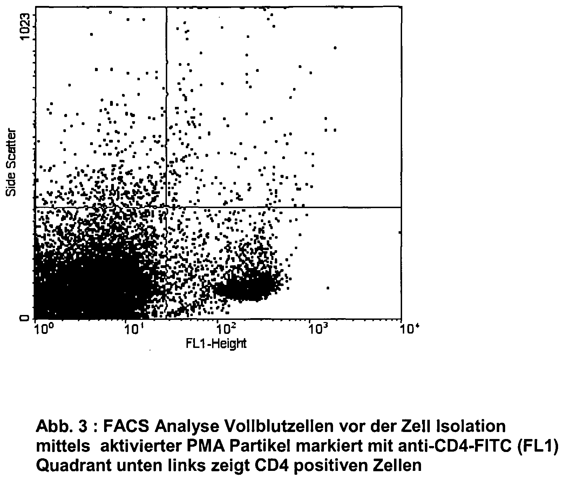

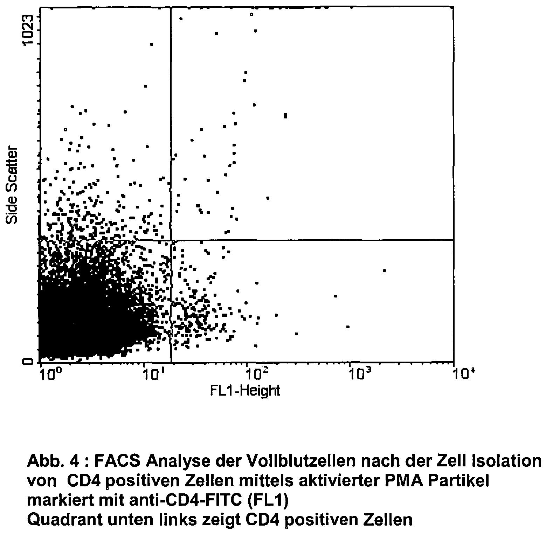

Spezifische Zellisolierung von CD4 positiven Zellen aus Vollblut
1. 4 ml anti-coaguliertes Ratten-Vollblut wird mit 1 ml (1 mg Partikel) funktionalisierter anti-CD4-PMA-Partikel Suspension gemischt.
2. 60 min bei Raumtemperatur wippend inkubieren.
3. Trennen der anti-CD4-PMA-Partikel (mit und ohne gebundene Zellen) aus dem Vollblut durch Filtration des Blut-Partikel-Gemisches mittels einer speziellen Kammer mit einem Zellsieb (40 µm).
4. Waschen der anti-CD4-PMA-Partikel mit 30 ml PBS (1% FCS)
5. Aufnahme der Partikel aus der Siebkammer in ca. 1 ml PBS, Vortexen (Mixen) für 15-20 Sekunden.
6. Entnahme einer Partikelprobe für mikroskopische Analyse (Abbildung 2).
7. FACS-Analytik der Leukozyten-Fraktion im Rattenvollblut vor und nach Behandlung mit den anti-CD4-PMA-Partikel (Abbildungen 3 und 4).

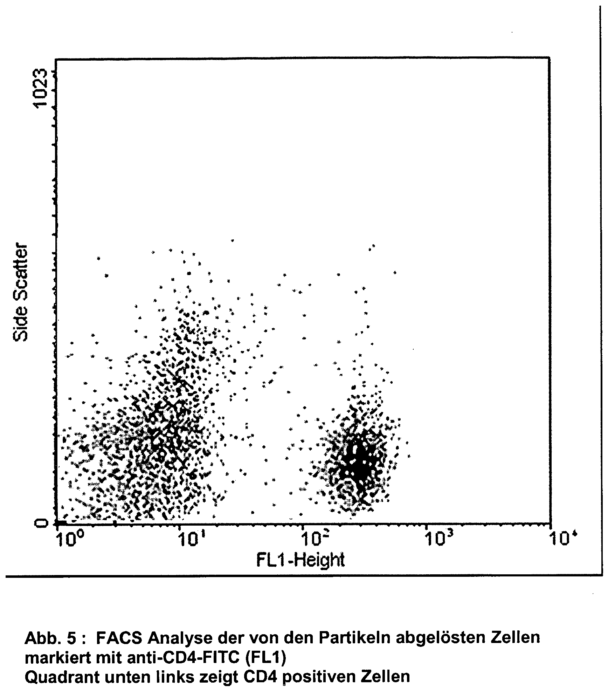

Ablösen und Qualifizierung der Zellen von den Partikeln.
1. anti-CD4-PMA-Partikel den anhaftenden Zellen werden durch vorsichtige Zentrifugation sedimentiert.
2. Aufnehmen des Sediments in PBS, 2 mM EDTA, 3 mM Mercaptoethanol und 20 U Papain.
3. Inkubation unter Mischen für 30 min.
4. Trennen von Zellen und Partikeln durch eine Filtration mittels einer speziellen Kammer mit einem Zellsieb (40 µm); Waschen der Partikel mit 30 ml PBS (1% FCS); Aufnahme der Partikel aus dem Zellsieb in ca. 1 ml PBS; Vortexen (Mixen) für 15-20 Sekunden und Wiederholen des Waschens.
5. Isolierte Lymphozytenfraktion durch Zentrifugation 350 x g aufkonzentrieren und mittels FACS analysiert (Abbildung 5).

### Ausführungsbeispiel 2 :

### Isolierung eines Proteins (IgG) aus einem Zell-Lysat

Ascites Antikörper (RIB 5/2) wurden mit Hilfe vom Millipore Montage Antibody Purification Kit (LSK2 ABG 20) nach Protokoll durchgeführt.

Die Aufreinigung wurde anschließend mit einem SDS Denaturierungs-Gel (10%) überprüft.

Kopplung der (Maus-IgG₂) Anti-CD4 Antikörper an die Polymethylmethcrylat (PMA)-Partikel
1. 1 ml PMA (Partikeldurchmesser = 40 µm +/- 10 µm; 10 mg/ml; COOH/PEG-COOH modifiziert,) zentrifugiert 2 min 3.000x g, Überstand verwerfen und in 1 ml 0,1M MES Puffer pH 6.3 aufnehmen.
2. 2 mg EDC und 2,4 mg N-Hydroxysuccinimid in 0,5 ml 0,1M MES Puffer pH 6,3 lösen und zu der PMA-Partikel-Suspension geben. 1 Stunde bei Raumtemperatur unter Rühren inkubieren (Aktivierung der Partikel).
3. PMA-Partikel separieren durch Zentrifugation und 2mal waschen mit 0,1M MES Puffer pH 6,3.
4. Aktivierte PMA-Partikel in 1 ml 0,1M MES Puffer pH 6.3 aufnehmen mit 100-150 µg Antikörper; Kopplungsreaktion bei Raumtemperatur für 16 Stunden (über Nacht).
5. Neutralisation freier Bindungsstellen durch Zugabe von 100 µl 1M Ethanolamine mit anschließender Inkubation für 1 Stunde.
6. Separieren mittels Zentrifugation und waschen der funktionalisierten PMA-Partikel mit PBS 3 mal.
7. Aufnahme der Maus-IgG-PMA-Partikel in 1 ml PBS pH 7,4 und Lagerung bei 4°C. Überprüfung der Antikörperkopplung mittels Goat-anti-Maus Antikörper PE markiert (Abbildung 1).

Spezifische Isolierung von Goat-anti-Maus-IgG aus einem Zell-Lysat
1. 1 ml Zelllysat (HepG2, 4x10⁶ ) werden mit 1 mg Goat-anti-Maus-IgG versetzt.
2. Dem Proteingemisch werden 0,1 ml (100µg Partikel) funktionalisierte Maus-IgG-PMA-Partikel Suspension zugegeben.
3. 60 min bei Raumtemperatur wippend inkubieren.
4. Trennen der Maus-IgG-PMA-Partikel (mit und ohne dem gebundenen Goat-anti-Maus-IgG) aus dem Zelllysat durch Filtration mittels einer speziellen Kammer mit einer Siebmembran (10 µm).
5. 3 x Waschen der PMA-Partikel mit je 5 ml PBS.

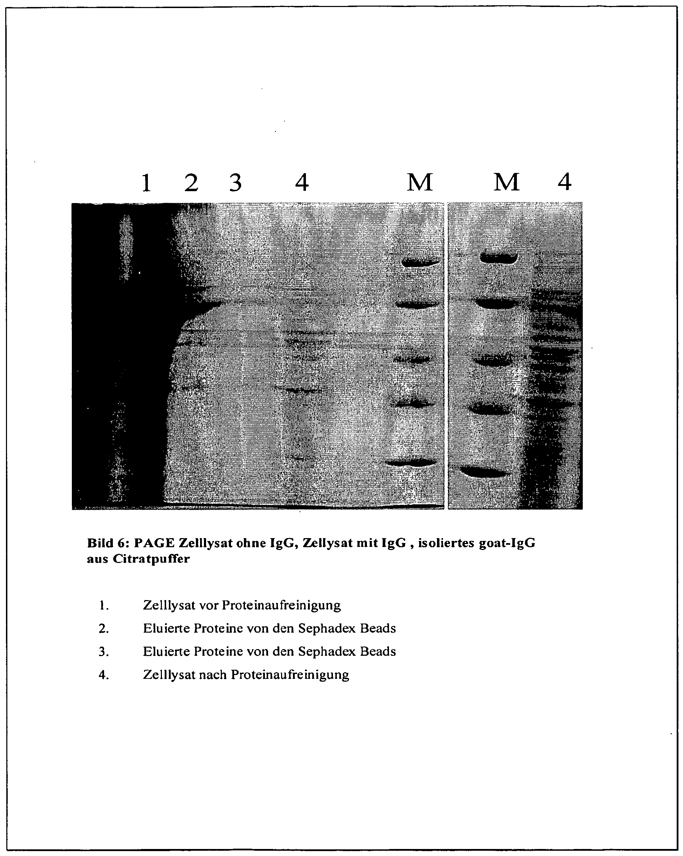

Ablösen und Qualifizierung des Proteins von den Partikeln.
1. Aufnahme der funktionalisierten PMA-Partikel mit den anhaftenden Proteinen aus der Siebkammer in 0,5 ml Citratpuffer pH 2,2; Inkubation für 30 min.
2. Abtrennung der Partikeln durch eine Filtration mittels einer speziellen Kammer mit einer Siebmembran (10 µm); Waschen der Partikel mit 2 ml Citratpuffer.
3. Abgelöste Proteine im Citratpuffer analysieren (Abbildung 6).

### Vergleichsbeispiel 1

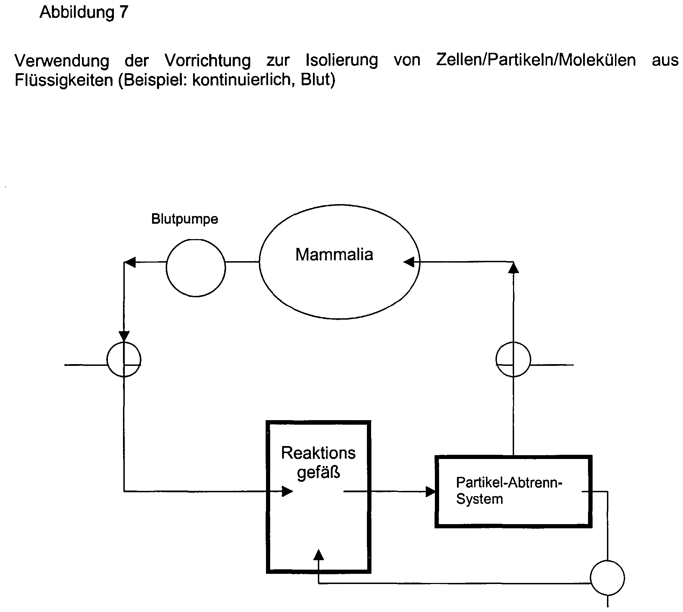

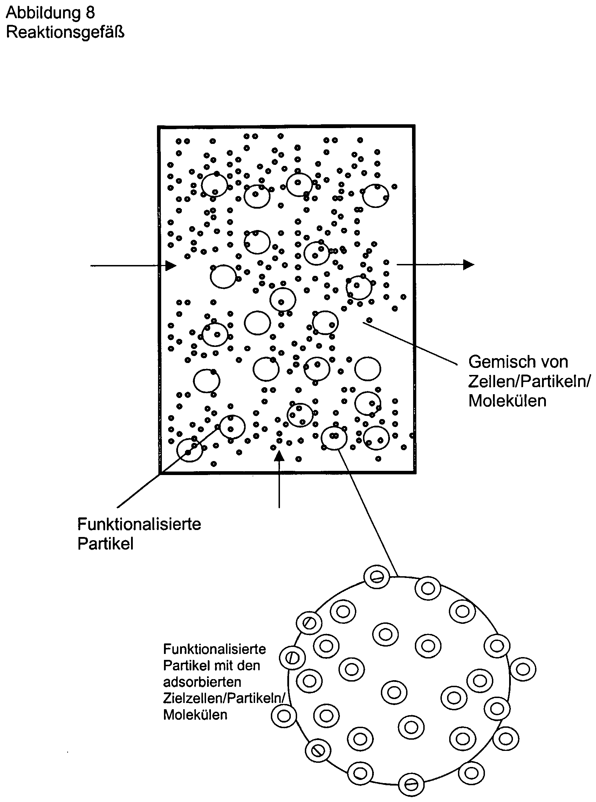

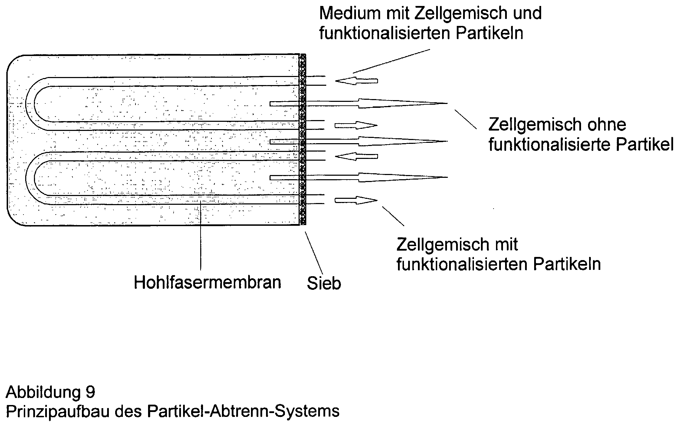

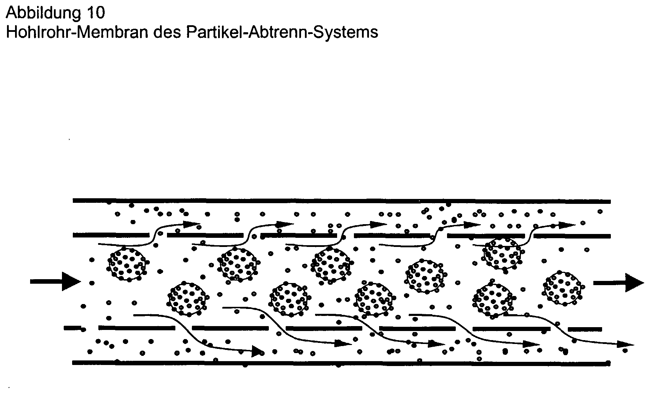

Die Abbildungen 7-11 zeigen Beispiele für die apparative Realisierung der Erfindung bzw. von Teilen davon. Abbildung 7 stellt ein System dar, das typischerweise für eine kontinuierliche Nutzung verwendbar ist. Gestrichelte Pfeile repräsentieren das Schlauchsystem, Pfeilspitzen markieren die Fließrichtung der Flüssigkeit: Ausgehend vom Organismus (Mammalia) wird über eine Blutpumpe und ein Ventil (in der Darstellung mit einem Kreis umgeben) Körperflüssigkeit, beispielsweise Blut, in ein Reakionsgefäß gepumpt bzw. geleitet, das funktionalisierte Partikel enthält, die im Gemisch mit Bestandteilen der Körperflüssigkeit, gegebenenfalls wechselwirken (Abbildung 8). Das Medium mit Zellgemisch und funktionalisierten Partikeln wird anschließend in ein Partikel-Abtrenn-System geleitet. Nach einem Beispiel wird das Medium mit Zellgemisch und funktionalisierten Partikeln über ein Sieb und über eine Hohlfasermembran geleitet und in ein Zellgemisch ohne funktionalisierte Partikel und in ein Zellgemisch mit funktionalisierten Partikeln aufgetrennt (Abbildung 9). Während das abgereicherte Zellgemisch ohne funktionalisierte Partikel abgeleitet über ein Ventil dem Organismus zugeführt wird, wird das Zellgemisch mit funktionalisierten Partikeln und die daran gebundenen Zielzellen/Biopartikel/Moleküle zum Reaktionsgefäß zurückgeführt (Abbildung 7). Abbildung 10 zeigt exemplarisch das Prinzip der Auftrennung innerhalb der Hohlfasermembran. Das Medium mit Zellgemisch und funktionalisierten Partikeln wird durch eine Hohlrohr-Membran geleitet, wobei die Membran mit einer Porengröße ausgestattet ist (gestrichelte Linie), die lediglich den Durchtritt des Zellgemischs, nun im wesentlichen die Zielzellen/Biopartikeln/Molekülen nicht mehr enthaltend, gestattet, während die funktionalisierten Partikel mit den abgetrennten Zielzellen/Biopartikeln/Molekülen im inneren des Hohlrohrsystems verbleiben.

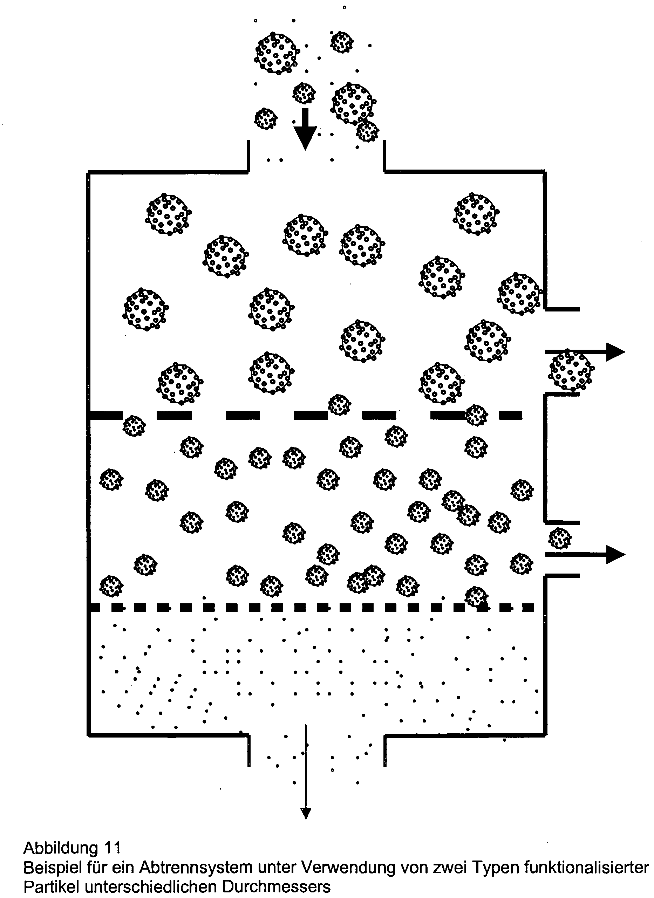

In Abbildung 11 ist im Detail ein entsprechendes Partikel-Abtrennsystem dargestellt, das für die Verwendung von zwei Typen funktionalisierter Partikel mit unterschiedlichem Durchmesser Verwendung finden kann.

## Patentansprüche

1. Vorrichtung zur Isolierung von Zellen aus Blut, bestehend aus
a. einem Reaktionsgefäß aus biokompatiblem Material, in dem sich ein Gemisch von Zellen befindet,
b. funktionalisierten, frei beweglichen Mikropartikeln mit einem Durchmesser von 50-500µm, die die gewünschten Zellen erkennen und binden,
c. einem Partikel-Abtrenn-System basierend auf einer oder mehrerer Membranen mit Poren, die Blutbestandteile, nicht aber die Mikropartikel passieren lassen, so dass die an die funktionalisierten Mikropartikel gebundenen Zellen von den ungebundenen Blutbestandteilen abtrennbar sind,
d. und diversen Schlauchsystemen, Membranen, Pumpen und Ventilen, um alle Handlungen der Isolierung, Vereinzelung, Bearbeitung und Abtrennung der Zellen vornehmen zu können.

2. Vorrichtung nach Anspruch 1, **dadurch gekennzeichnet, dass**
- die spezifischen Zellen somatische Zellen, Viren, Bakterien und/oder Protozoen sind; und
- die Zell-Separation durch die Verwendung weiterer funktionalisierter Mikropartikel kombiniert wird, wobei sich insbesondere die kombinierten funktionalisierten Mikropartikel in ihrer Größe unterscheiden und dass vorzugsweise über (einen) zusätzliche(n) Liganden eine Zell-stimulierende Funktion für lebende Zellen erfolgen kann oder dass vorzugsweise über den/die zusätzlichen Liganden mindestens zwei unterschiedliche Zelltypen separiert werden; und/oder
- diese funktionalisierten Mikropartikel mehr als einen spezifischen Liganden tragen und somit zwei und mehr unterschiedliche Zellen auf dem Mikropartikel spezifisch adsorbiert werden.

3. Vorrichtung nach einem der Ansprüche 1 - 2, **dadurch gekennzeichnet, dass**
- die funktionalisierten Mikropartikel in dem Blut suspendiert und frei beweglich sind; und/oder
- die Mikropartikel durch mechanische Einwirkung in Suspension gehalten werden; und
- das Blut aus dem mit den Mikropartikeln versehenen Reaktionsraum durch fraktionierte Filtration von den Mikropartikeln befreit wird; und
- für die Filtration eine oder mehrere Membranen verwendet werden, die sich durch Poren mit einem Durchmesser auszeichnen, die alle nicht spezifisch adsorbierten Bestandteile passieren lassen, nicht aber die funktionalisierten Mikropartikel mit den spezifisch adsorbierten Zellen; und
- Flach- oder Hohlrohr-Membranen zur Abtrennung eingesetzt werden, wobei der Porendurchmesser kleiner ist als die verwendeten Mikropartikel; und
- das Reaktionsgefäß für die weitere Bearbeitung der isolierten Zellen verwendet wird.

4. Verfahren zur Abtrennung von Zellen aus einer Blutprobe, **dadurch gekennzeichnet, dass** Blut mit den funktionalisierten Mikropartikeln der Vorrichtung aus Anspruch 1 in Kontakt gebracht werden, und die abzutrennenden Zellen, welche spezifisch an die funktionalisierten Mikropartikel gebunden haben, über eine oder mehrere Membranen mit Porengrößen, die alle Zellen, nicht aber bestimmte funktionalisierten Mikropartikel, passieren lassen, abgetrennt werden.

5. Verfahren nach Anspruch 4 zur Abtrennung von Zellen, **dadurch gekennzeichnet, dass** spezifische Bindung durch einen Liganden natürlichen oder synthetischen Ursprungs erfolgt, insbesondere dass der Ligand ein Antikörper, ein Antikörperfragment, ein Peptid, ein Polypeptid, ein Glykopeptid, ein Rezeptor, ein Steroid, ein Hormon, ein Mitogen, ein Antigen, ein Superantigen, ein Wachstumsfaktor, ein Zytokin, ein Lektin, ein Virusprotein, ein Adhäsionsmolekül oder ein Chemokin ist.

6. Verfahren nach Anspruch 5, **dadurch gekennzeichnet, dass**
- der Ligand kovalent oder nicht-kovalent auf dem Mikropartikel gebunden ist; und
- als feste Träger biokompatible Polymere verwendet werden; und
- die Liganden an vorzugsweise Sepharose, Polytactide, und Alginat gekoppelt werden; und
- eine kontinuierliche oder diskontinuierliche Separation von Zellen vorliegt; und
- die Reaktionsgefäße mit unterschiedlich funktionalisierten Mikropartikeln und Trennmembranen mit definierten Porendurchmesser als einzelne Module in beliebiger Anzahl in Reihe geschaltet werden können; und/oder
- für die kontinuierliche Separation die funktionalisierten Mikropartikel in das Reaktionsgefäß eingebracht werden, das mit dem Zellen enthaltendem Blut durchströmt wird; und/oder
- die Zellen von den Mikropartikeln durch Anwendung von Puffern abgelöst werden; und/oder
- die Zellfreisetzung und bei Bedarf die Freisetzung weiterer Zellen im gleichen Reaktionsgefäß erfolgt; und
- die Zellen durch die gleiche Membranfiltration von der Mikropartikelsuspension abgetrennt wird, wie sie für die Flüssigkeitsabtrennung verwendet wird;

7. Vorrichtung nach einem der Ansprüche 1 - 3 zur Isolierung von Zellen aus Blut, mit
a. einem Reaktionsgefäß aus biokompatiblem Material, in dem sich ein Gemisch von Zellen befindet,
b. funktionalisierten Mikropartikeln, die die gewünschten Zellen erkennen und binden, wobei die Mikropartikel frei beweglich und von dem Reaktionsgefäß zu einem Partikel-Abtrenn-System leitbar sind,
c. einem Partikel-Abtrenn-System basierend auf einer oder mehrerer Membranen/Siebe mit Poren, die die ungebundenen Blutbestandteile, nicht aber die Mikropartikel passieren lassen, für
das Auftrennen eines Mediums mit einem Zellgemisch und Mikropartikeln durch hydrostatischen Duck in ein Zellgemisch mit Mikropartikeln und in ein Zellgemisch ohne Mikropartikel, ,
d. einer Sieb/Hohlfasermembran, wobei die Membran mit einer Porengröße ausgestattet ist, die lediglich den Durchtritt des Zellgemischs ohne Mikropartikel gestattet,
e. gegebenenfalls einer Pumpe, um Blut über ein Ventil in das Rektionsgefäß zu pumpen/leiten,
f. einem Ventil, über das das Blut in das Reaktionsgefäß gepumpt/geleitet wird,
g. einem Ventil, über das das Zellgemisch ohne Mikropartikel von den Partikel-Abtrennsystem abgeleitet wird,
h. einem Schlauchsystem zum (A) Leiten oder Pumpen von Blut mit einem Zellgemisch über ein Ventil in ein Reaktionsgefäß, das funktionalisierte Mikropartikel enthält, und zum (B) Leiten des Mediums mit Zellgemisch und funktionalisierten Mikropartikeln von dem Reaktionsgefäß über ein Sieb mit einer Hohlfasermembran zum Auftrennen des Mediums in ein Zellgemisch ohne funktionalisierte Mikropartikel und in ein Zellgemisch mit funktionalisierten Mikropartikeln und zum (C) Ableiten des aufgetrennten Zellgemischs ohne funktionalisierte Mikropartikel über ein Ventil und zum (D) Zurückführen des aufgetrennten Zellgemischs mit den funktionalisierten Mikropartikeln und den daran gebundenen Zellen zum Reaktionsgefäß oder zum Ableiten des Zellgemischs mit den funktionalisierten Mikropartikeln und den daran gebundenen Zellen für analytische oder präparative Zwecke,
i. und Schlauchsystemen, Membranen, Pumpen und Ventilen, um alle Handlungen der Isolierung, Vereinzelung, Bearbeitung und Abtrennung der Zellen vornehmen zu können.

8. Verfahren nach einem der Ansprüche 4-6 zur Abtrennung von Zellen aus Blut, **dadurch gekennzeichnet, dass**
- über ein Ventil Blut mit Zellen in ein biokompatibles Reaktionsgefäß gepumpt oder geleitet wird, das funktionalisierte Mikropartikel enthält, die im Gemisch mit Bestandteilen des Blutes ggf. wechselwirken,
- das Medium mit Zellgemisch und funktionalisierten Mikropartikeln von dem Reaktionsgefäß in ein Partikel-Abtrenn-System mit Sieb und Hohlfasermembranen geleitet wird, wobei die Membranen mit einer Porengröße ausgestattet sind, die lediglich den Durchtritt des Zellgemisches ohne funktionalisierte Mikropartikel gestattet, so dass das Medium mit Zellgemisch und funktionalisierten Mikropartikeln durch hydrostatischen Druck über Siebe und die Hohlfasermembranen in ein Zellgemisch ohne funktionalisierte Mikropartikel und in ein Zellgemisch mit funktionalisierten Mikropartikeln aufgetrennt wird,
- das aufgetrennte Zellgemisch ohne funktionalisierte Mikropartikel von dem Mikropartikel-Abtrenn-System über ein Ventil abgeleitet wird, und
- das Zellgemisch mit funktionalisierten Mikropartikeln und die daran ggf. gebundenen Zielzellen zum Reaktionsgefäß zurückgeführt wird oder für analytische oder präparative Zwecke abgeleitet wird.

9. Vorrichtung nach einem der Ansprüche 1 - 3 und 7 zur ex-vivo-Behandlung von Blut in einem extra-korporalen Kreislauf.

10. Verfahren nach einem der Ansprüche 4-6 und 8 zur ex-vivo-Behandlung von Blut in einem extra-korporalen Kreislauf.

11. Vorrichtung nach einem der Ansprüche 1 - 3 und 7 zur Reinigung, An- oder Abreicherung von Zellen für Diagnostik.

12. Verfahren nach einem der Ansprüche 4-6 und 8 zur Reinigung, An- oder Abreicherung von Zellen für Diagnostik.

## Claims

1. A device for isolating cells from blood, consisting of
a. a reaction vessel made of biocompatible material containing a mixture of cells
b. functionalised, free-moving microparticles with a diameter of 50-500µm, which recognise and bind the desired cells,
c. a particle separation system based on one or more membranes with pores that allow blood components but not the microparticles to pass, so that the cells bound to the functionalised microparticles are separable from the unbound blood components,
d. and various hose systems, membranes, pumps and valves to perform all acts of isolation, separation, processing and separation of the cells.

2. A device according to claim 1, **characterized in that**
- the specific cells are somatic cells, viruses, bacteria and/or protozoa; and
- the cell separation is combined by the use of further functionalised microparticles, whereby in particular the combined functionalised microparticles differ in size and that preferably via (an) additional ligand(s) a cell-stimulating function for living cells can be performed or that preferably via the additional ligand(s) at least two different cell types are separated; and/or
- these functionalized microparticles carry more than one specific ligand and thus two and more different cells are specifically adsorbed on the microparticle.

3. A device according to any one of claims 1 - 2, **characterized in that**
- the functionalised microparticles are suspended and freely movable in the blood; and/or
- the microparticles are kept in suspension by mechanical action; and
- the blood from the reaction chamber containing the microparticles is freed from the microparticles by fractional filtration; and
- one or more membranes are used for filtration, **characterised by** pores with a diameter that allows all non-specifically adsorbed components to pass through, but not the functionalised microparticles containing the specifically adsorbed cells; and
- flat or hollow tube membranes are used for separation, where the pore diameter is smaller than the microparticles used; and
- the reaction vessel is used for further processing of the isolated cells.

4. Method for separating cells from blood, **characterized in that** blood is brought into contact with the functionalized microparticles of the device of claim 1, and the cells to be separated, which have bound specifically to the functionalized microparticles, are separated via one or more membranes with pore sizes which allow all cells, but not certain functionalized microparticles, to pass.

5. Method according to claim 4 for the separation of cells, **characterized in that** specific binding takes place by a ligand of natural or synthetic origin, in particular that the ligand is an antibody, an antibody fragment, a peptide, a polypeptide, a glycopeptide, a receptor, a steroid, a hormone, a mitogen, an antigen, a superantigen, a growth factor, a cytokine, a lectin, a viral protein, an adhesion molecule or a chemokine.

6. Method according to claim 5, **characterized in that**
- the ligand is covalently or non-covalently attached to the microparticle; and
- biocompatible polymers are used as solid carriers; and
- the ligands are coupled to preferably sepharose, polyvinyl acrylate, polymethyl methacrylate, polymethyl acrylate, polylactides, alginate, glass, silicate; and
- a continuous or discontinuous separation of cells; and
- the reaction vessels with differently functionalised microparticles and separation membranes with defined pore diameters can be connected in series as individual modules in any number; and/or
- for continuous separation, the functionalised microparticles are introduced into the reaction vessel through which the blood containing the cells flows; and/or
- the cells are detached from the microparticles by applying special buffers; and/or
- the cell release and, if necessary, the release of further cells takes place in the same reaction vessel; and
- the cells are separated from the microparticle suspension by the same membrane filtration as used for liquid separation.

7. a device according to any one of claims 1 - 3 for the isolation of cells from blood, comprising
a. A reaction vessel made of biocompatible material containing a mixture of cells
b. Functionalized microparticles which recognize and bind the desired cells, said microparticles being freely movable and directable from the reaction vessel to a particle separation system,
c. a particle separation system based on one or more membranes/screens with pores that allow the unbound blood components but not the microparticles to pass, for the separation of a medium with a cell mixture and microparticles by hydrostatic pressure into a cell mixture with microparticles and a cell mixture without microparticles,
d. A screen/hollow fibre membrane, the membrane having a pore size that allows only the passage of the cell mixture without microparticles,
e. a pump, if necessary, to pump/direct blood via a valve into the reaction vessel,
f. A valve through which the blood is pumped/directed into the reaction vessel
g. A valve through which the cell mixture without microparticles is discharged from the particle separation system,
h. a tubing system for (A) conducting or pumping blood with a cell mixture via a valve into a reaction vessel containing functionalized microparticles, and for (B) passing the medium with cell mixture and functionalized microparticles from the reaction vessel through a screen with a hollow fiber membrane for separating the medium into a cell mixture without functionalized microparticles and a cell mixture with functionalized microparticles and for (C) discharging the separated cell mixture without functionalized microparticles via a valve and for (D) returning the separated cell mixture with the functionalized microparticles and the cells bound thereto to the reaction vessel or for (E) discharging the cell mixture with the functionalized microparticles and the cells bound thereto for analytical or preparative purposes,
i. and tubing systems, membranes, pumps and valves to perform all acts of isolation, separation, processing and separation of the cells.

8. Method according to any one of claims 4 - 6 for separating cells from blood, **characterized in that**
- via a valve, blood with cells is pumped or directed into a biocompatible reaction vessel containing functionalised microparticles that may interact with components of the blood,
- the medium with cell mixture and functionalised microparticles is passed from the reaction vessel into a particle separation system with screen and hollow fibre membranes, the membranes being provided with a pore size which only permits the passage of the cell mixture without functionalised microparticles, so that the medium with cell mixture and functionalised microparticles is separated by hydrostatic pressure via screens and the hollow fibre membranes into a cell mixture without functionalised microparticles and into a cell mixture with functionalised microparticles,
- the separated cell mixture without functionalised microparticles is discharged from the microparticle separation system via a valve, and
- the cell mixture with functionalised microparticles and the target cells possibly bound to them is returned to the reaction vessel or is discharged for analytical or preparative purposes.

9. Apparatus according to any one of claims 1 - 3 and 7 for the ex vivo treatment of blood in an extracorporeal circulation.

10. Method according to any one of claims 4 - 6 and 8 for the ex vivo treatment of blood in an extracorporeal circulation.

11. Apparatus according to any one of claims 1 - 3 and 7 for cleaning, enriching or depleting cells for diagnostics.

12. Method according to any one of claims 4 - 6 and 8 for cleaning, enrichment or depletion of cells for diagnostics.

## Revendications

1. Dispositif d'isolement de cellules du sang, constitué de :
a. une éprouvette en matériau biocompatible dans laquelle se trouve un mélange de cellules ;
b. microparticules fonctionnalisées et mobiles d'un diamètre de 50 à 500 µm identifiant et liant les cellules souhaitées ;
c. un système de séparation de particules fonctionnant à l'aide d'une ou de plusieurs membrane(s) présentant des pores ; ceux-ci laissent passer les composants sanguins, mais pas les microparticules, ce qui permet de séparer les cellules liées aux microparticules fonctionnalisées des composants sanguins non liés ; et
d. divers tuyaux, membranes, pompes et soupapes permettant de réaliser toutes les opérations liées à l'isolement, au traitement et à la séparation des cellules.

2. Dispositif au sens de la revendication 1, **caractérisé par le fait que** :
- les cellules souhaitées sont des cellules somatiques, des virus, des bactéries et/ou des protozoaires ; et
- la séparation des cellules est complétée par l'utilisation de microparticules fonctionnalisées supplémentaires, ces microparticules fonctionnalisées combinées présentant notamment des tailles variables, de façon à ce que, de préférence, les cellules vivantes puissent être stimulées par l'intermédiaire d'un ou plusieurs ligand(s) supplémentaire(s) ou au moins deux types de cellule différents puissent être séparés par l'intermédiaire du/des ligand(s) supplémentaire(s) ; et/ou
- ces microparticules fonctionnalisées portent plusieurs ligands spécifiques, donc deux cellules différentes ou plus parmi les cellules souhaitées se trouvant sur une microparticule sont adsorbées.

3. Dispositif au sens de la revendication 1 ou 2, **caractérisé par le fait que** :
- les microparticules fonctionnalisées sont en suspension et mobiles dans le sang ; et/ou
- les microparticules sont maintenues en suspension par action mécanique ; et
- les microparticules sont éliminées du sang de la chambre de réaction contenant les microparticules par filtration fractionnée ; et
- la filtration s'effectue à l'aide d'une ou plusieurs membrane(s) présentant des pores dont le diamètre laisse passer tous les composants non souhaités qui ont été adsorbés, mais pas les microparticules fonctionnalisées portant les cellules souhaitées adsorbées ; et
- des membranes à tuyaux plats ou creux sont employées pour la séparation, le diamètre de leurs pores étant inférieur à la taille des microparticules utilisées ; et
- l'éprouvette est utilisée pour la suite du traitement des cellules isolées.

4. Procédé de séparation de cellules d'un échantillon de sang, **caractérisé par le fait que** le sang est mis en contact avec les microparticules fonctionnalisées du dispositif de la revendication 1 et que les cellules à séparer, qui se sont liées aux microparticules fonctionnalisées, sont séparées par l'intermédiaire d'une ou de plusieurs membrane(s) dont la taille des pores laisse passer toutes les cellules, mais pas certaines microparticules fonctionnalisées.

5. Procédé au sens de la revendication 4, servant à la séparation de cellules et **caractérisé par le fait qu'**un ligand d'origine naturelle ou synthétique crée une liaison spécifique, en particulier **par le fait que** le ligand est un anticorps, un fragment d'anticorps, un peptide, un polypeptide, un glycopeptide, un récepteur, un stéroïde, une hormone, un mitogène, un antigène, un superantigène, un facteur de croissance, une cytokine, une lectine, une protéine virale, une molécule d'adhérence ou une chimiokine.

6. Procédé au sens de la revendication 5, **caractérisé par le fait que** :
- le ligand est lié à une microparticule de façon covalente ou non covalente ; et
- des polymères biocompatibles sont utilisés comme supports solides ; et
- les ligands se lient de préférence au gel Sepharose, aux polylactides et à l'alginate ; et
- les cellules sont séparées de manière continue ou discontinue ; et
- un nombre quelconque d'éprouvettes **caractérisées par** des microparticules fonctionnalisées différentes et des membranes de séparation avec des pores au diamètre défini peuvent être utilisées comme des modules individuels et montées en série ; et/ou
- les microparticules fonctionnalisées sont introduites dans l'éprouvette traversée par le sang contenant des cellules à des fins de séparation continue ; et/ou
- les cellules sont remplacées par les microparticules via l'utilisation de tampons ; et/ou
- la libération des cellules et, si nécessaire, la libération d'autres cellules s'effectuent dans la même éprouvette ; et
- les cellules sont séparées des microparticules en suspension via la même filtration par membrane que celle utilisée pour la séparation des liquides ;

7. dispositif au sens de la revendication 1, 2 ou 3, servant à l'isolement de cellules du sang et constitué de :
a. une éprouvette en matériau biocompatible dans laquelle se trouve un mélange de cellules ;
b. des microparticules fonctionnalisées identifiant et liant les cellules souhaitées, ces microparticules étant mobiles et pouvant être dirigées de l'éprouvette vers un système de séparation de particules ;
c. un système de séparation de particules fonctionnant à l'aide d'un(e) ou de plusieurs membrane(s)/tamis présentant des pores ; ceux-ci laissent passer les composants sanguins non liés, mais pas les microparticules. Il sert à séparer, via pression hydrostatique, un fluide contenant un mélange de cellules et des microparticules en un mélange de cellules avec microparticules et un mélange de cellules sans microparticules
d. un tamis/une membrane à fibres creuses dont la taille des pores laisse uniquement passer le mélange de cellules sans microparticules ;
e. une pompe, le cas échéant, pour pomper/diriger le sang dans l'éprouvette par l'intermédiaire d'une soupape ;
f. une soupape permettant de pomper/diriger le sang dans l'éprouvette ;
g. une soupape permettant d'évacuer le mélange de cellules sans microparticules du système de séparation de particules ;
h. un système de tuyaux pour (A) diriger ou pomper, par l'intermédiaire d'une soupape, le sang contenant un mélange de cellules dans une éprouvette contenant des microparticules fonctionnalisées, pour (B) diriger le fluide contenant un mélange de cellules et des microparticules fonctionnalisées de l'éprouvette vers un tamis pourvu d'une membrane à fibres creuses afin que le fluide soit séparé en un mélange de cellules sans microparticules fonctionnalisées et un mélange de cellules avec microparticules fonctionnalisées, pour (C) évacuer le mélange de cellules séparé sans microparticules fonctionnalisées par l'intermédiaire d'une soupape et pour (D) refouler dans l'éprouvette le mélange de cellules séparé contenant les microparticules fonctionnalisées et les cellules qui y sont liées ou pour évacuer le mélange de cellules contenant les microparticules fonctionnalisées et les cellules qui y sont liées à des fins d'analyse ou de préparation ;
i. tuyaux, membranes, pompes et soupapes permettant de réaliser toutes les opérations liées à l'isolement, au traitement et à la séparation des cellules.

8. Procédé au sens de la revendication 4, 5 ou 6, servant à la séparation de cellules du sang et **caractérisé par le fait que** :
- une soupape permet de pomper ou diriger du sang contenant des cellules dans une éprouvette biocompatible contenant des microparticules fonctionnalisées interagissant éventuellement avec les composants sanguins avec lesquels elles sont mélangées ;
- le fluide contenant un mélange de cellules et des microparticules fonctionnalisées est dirigé de l'éprouvette vers un système de séparation de particules pourvu d'un tamis et de membranes à fibres creuses, les pores de ces membranes laissant passer uniquement le mélange de cellules sans microparticules fonctionnalisées de façon à ce que le fluide contenant un mélange de cellules et des microparticules fonctionnalisées soit séparé, via pression hydrostatique et par l'intermédiaire des tamis et des membranes à fibres creuses, en un mélange de cellules sans microparticules fonctionnalisées et un mélange de cellules avec microparticules fonctionnalisées ;
- le mélange de cellules séparé sans microparticules fonctionnalisées est évacué du système de séparation de particules par l'intermédiaire d'une soupape ; et
- le mélange de cellules séparé avec microparticules fonctionnalisées et les cellules cibles qui y sont éventuellement liées sont refoulés dans l'éprouvette ou évacués à des fins d'analyse ou de préparation.

9. Dispositif au sens des revendications 1, 2, 3 et 7, servant au traitement ex vivo du sang dans un circuit extra-corporel.

10. Procédé au sens des revendications 4, 5, 6 et 8, servant au traitement ex vivo du sang dans un circuit extra-corporel.

11. Dispositif au sens des revendications 1, 2, 3 et 7, servant au nettoyage, à l'enrichissement ou à l'appauvrissement de cellules à des fins de diagnostic.

12. Procédé au sens des revendications 4, 5, 6 et 8, servant au nettoyage, à l'enrichissement ou à l'appauvrissement de cellules à des fins de diagnostic.
